Europäisches Patentamt

**European Patent Office**

Office européen des brevets

(11) Publication number: **0 254 457**
**A1**

## (12) EUROPEAN PATENT APPLICATION

(21) Application number: **87306108.9**

(22) Date of filing: **10.07.87**

(51) Int. Cl.⁴: **G01N 33/52**

(30) Priority: **10.07.86 US 884321**

(43) Date of publication of application:
**27.01.88 Bulletin 88/04**

(84) Designated Contracting States:
**CH DE FR GB LI**

(71) Applicant: **EASTMAN KODAK COMPANY**
**343 State Street**
**Rochester New York 14650(US)**

(72) Inventor: **Columbus, Richard Lewis EASTMAN**
**KODAK COMPANY**
**Patent Department 343 State Street**
**Rochester New York 14650(US)**
Inventor: **Palmer, Harvey John EASTMAN**
**KODAK COMPANY**
**Patent Department 343 State Street**
**Rochester New York 14650(US)**

(74) Representative: **Davis, Ian Ellison et al**
**Kodak Limited Patent Department Headstone**
**Drive**
**Harrow Middlesex HA1 4TY(GB)**

(54) **Analytical element having spread control zone.**

(57) An analytical element for determining analytes in aqueous liquids, such as biological fluids, has therein a spread control zone comprising a polymeric material that is naturally soluble in water. The polymer dissolves upon contact with the aqueous sample, thereby increasing the viscosity of the aqueous sample such that the spread of the aqueous sample in the element is reduced. The element is used by contacting, in the presence of an indicator composition for providing a detectable change in response to the analyte, a finite area of the element with the aqueous sample and determining the presence or amount of the analyte as a function of the detectable change in the indicator composition.

EP 0 254 457 A1

## ANALYTICAL ELEMENT HAVING SPREAD CONTROL ZONE

This invention relates to clinical chemistry and to elements and methods useful for the determination of analytes in aqueous liquids.

An increasingly large number of analytical tests, procedures, and analyses (i.e., assays) must be performed each day on many kinds of aqueous liquid samples, including biological liquids, such as blood, serum, urine, cerebrospinal fluid, and the like. To effectively handle and meet critical laboratory needs, "dry chemistry" analytical elements used in these analyses should require minimal operator involvement and provide rapid, accurate and reproducible results.

A number of types of analyses can be performed using "dry chemistry" analytical elements. These include end-point analyses, such as the glucose assay described in U.S. Patent 4,098,574, rate assays as described in U.S. Patent 4,547,465 and heterogeneous immunoassays involving separation in the element of the bound and unbound labeled ligands, and homogeneous immunoassays, in which a labeled ligand exhibits a detectable difference when it is bound to a receptor. Uniform spreading in the element of the sample liquid is highly desirable, particularly when accurate quantitative results are desired in the detection of an analyte in the sample. Such uniform spreading can be obtained through the inclusion of spreading or transport zones, which may or may not contain other analytes (such as labelled ligand analogs, e.g., enzyme-labeled antigens, receptors, e.g., immobilized antibodies, or indicator compositions, e.g., enzyme substrates). Such spreading or transport zones are well known in the art, and include, for example, fibrous spreading layers and non-fibrous isotropically porous spreading layers. Such spreading or transport zones are described, for example, in U.S. Patents 3,992,158 and 4,258,001.

As the liquid sample spreads laterally in the layers of multilayer analytical elements, labeled antigen or small molecules, such as enzyme substrates, become diluted and can even be washed away from the area where the signal is read. Therefore, it is desirable to minimize the spread and particularly the lateral spread of the sample liquid in the layers of a multilayer analytical element. It may also be desirable in some immunoassays to slow down the lateral spread of the sample to give the competitive immunological reaction an opportunity to be adequately established before horizontal separation occurs, although enough lateral spreading must occur to allow separation of the bound and free labelled ligand.

Prior to the present invention, lateral spread could only be controlled through the use of surfactants or by adjusting the microporous structure or pore size distribution in the spreading layer of an analytical element. One problem is that surfactants can simultaneously alter the fluid flow behavior in the spreading layer in competing ways through their effects on surface tension and wettability. For example, increasing wettability will increase the spreading rate, while the simultaneous decrease in surface tension will decrease the spreading rate. Thus, the net effect can be the opposite to that originally desired. Furthermore, small amounts of surfactant can produce dramatic changes in flow behavior. Thus, it is difficult to control flow behavior with surfactants alone. Another problem is that decreasing the porosity of the spreading layer of an element can result in a loss of uniformity in the spreading of the sample and is limited in its application because too great a decrease in porosity can detrimentally impede the transport in the spreading layer of larger molecules and cells often found in the liquid samples.

The objective of the present invention is to achieve control over the lateral spread of aqueous liquid samples while avoiding the problems caused by surfactants and decreased pore size of the spread layer. That objective is achieved with the present invention which provides an analytical element comprising a spread control zone having a polymeric material that is naturally soluble in water. The polymeric material dissolves upon contact with the aqueous sample, increasing the viscosity of the aqueous sample such that the spread of the aqueous sample in the element is reduced. The transport of large molecules or cells is not affected. Larger pore sizes can be used for more uniform spreading and compatibility with whole blood. Protein bias due to the effect of proteins on spreading time and absorption rate can be decreased.

The invention also includes a method for the determination of an analyte component in an aqueous sample using an analytical element, comprising the steps of: in the presence of an indicator composition for providing a detectable change in response to said analyte, contacting a finite area of the element with said aqueous sample; and determining the presence or amount of said analyte as a function of the detectable change in said indicator composition, the spread of said aqueous sample in the element being controlled by contacting the aqueous sample with a polymeric material that is naturally soluble in water, in such a manner that the polymeric material in contact with the aqueous sample is dissolved, whereby the viscosity of the aqueous sample is increased and the spreading of the aqueous sample in the element is reduced.

The present invention will now be described by way of example with reference to the accompanying drawings in which:-

FIG. 1 represents a multilayer element of the invention having a spreading zone and a spread control zone that are separate layers of the element.

FIG 2 represents an alternate embodiment having spreading and spread control zones in separate layers.

FIG. 3 represents a multilayer element of the invention having a spreading zone and a spread control zone that are contained in a single layer of the element.

The element of the invention can be used to determine, either quantitatively or qualitatively, a number of analytes in an aqueous liquid, such as a biological fluid (e.g., whole blood, serum, plasma, urine, spinal fluid, suspensions of human or animal tissue, feces, saliva, lymphatic fluid, and the like). The elements of the invention can be used to test for various analytes found in biological fluids, such as glucose, aspartate aminotransferase, alanine aminotransferase and lactate dehydrogenase, and ligands such as therapeutic drugs (e.g., diphenylhydantoin, phenobarbital, theophylline, digoxin, gentamicin, quinidine, propanolol, tobramycin, lidocaine, procainamide, and the like), natural or synthetic steroids (e.g., cortisol, aldosterone, testosterone, progesterone, estriol, etc.), hormones (e.g., thyroid hormones, peptide hormones, insulin, etc.), proteins (e.g., albumin, IgG, IgM, etc.), antigens, antibodies, and other species that will naturally react with a receptor.

The spread control zone of the invention comprises a polymeric material that is naturally soluble in water. By "naturally soluble in water," it is meant that upon contact with water, the polymeric material becomes a homogeneous liquid or solution. Materials that merely absorb water or swell upon contact with water are not included within the scope of materials that are "naturally soluble in water." Such materials are more properly classified as water-swellable or water-dispersible. The polymers must be naturally soluble in water as opposed to insoluble, water-swellable or only water dispersible in order to increase the solution viscosity. An advantage of using these naturally soluble polymers is that they can easily be coated onto a support or another layer, or into a spreading layer by standard coating methods using standard coating solvents.

Polymeric materials naturally soluble in water that are useful in the present invention dissolve rapidly at least to the extent that a 0.5-25 $\mu$m thick layer of the material will dissolve in less than 5 minutes when contacted on one side of the layer with water below 40°C. Especially preferred polymeric materials will dissolve in less than 120 seconds when contacted on one side of the layer with water at 37°C. The water-soluble polymeric materials of the invention dissolve upon contact with the aqueous sample, thereby increasing the viscosity of the aqueous sample such that the spread of the sample in the element is reduced. The polymeric materials are preferably chosen so that this increase in viscosity is by a factor of at least 2-20. Examples of such polymer materials that are useful in the present invention include hydroxypropyl cellulose (MW = 60,000-1,000,000), hydroxyethyl cellulose (MW = 100,000-1,000,000), carboxymethyl cellulose, and carboxypropyl cellulose. Other useful polymers include unhardened gelatin, poly(acrylamide), poly(vinylpyrrolidone) and poly(vinylalcohol) or any mixtures or copolymers of this second group.

The spreading zone of the element has a suitable porosity for accommodating a test sample (e.g., 1 to 200 $\mu$l), diluted or undiluted. Useful spreading zones can be prepared from paper, porous particulate structures, porous polymeric films, cellulose, wood, glass fibers, woven and nonwoven fibrous fabrics (synthetic and nonsynthetic) and the like. Materials and processes for making such spreading zones are well known in the art. Useful spreading zones are described in, for example, U.S. Patents 4,292,272; 3,992,158; 4,258,001; and 4,430,436 along with Japanese Patent Publication 57(1982)-101760, the disclosures of which are incorporated herein by reference in their entirety.

One preferred spreading zone comprises organo-polymeric particles and a polymeric adhesive, as described in the above-referenced U.S. Patent 4,258,001.

The particles can be composed of a wide variety of organic polymers, including both natural and synthetic polymers, having the requisite properties. Preferably, however, they are composed of one or more addition polymers formed from one or more ethylenically unsaturated polymerizable monomers, such as addition homopolymers of single monomers or copolymers formed from two or more of such monomers. These polymers can be prepared by any of a variety of conventional polymerization methods (e.g. solution, emulsion, dispersion, suspension, etc.). If desired, although the invention is not so limited, the particular polymer can contain one or more reaction sites to link various interactive compositions to the particles.

The polymeric adhesive of U.S. Patent 4,258,001 bonds the organo-polymeric particles to one another to provide a coherent, three-dimensional lattice in the spreading layer. The details of this adhesive are provided in U.S. Patent 4,258,001 noted above. Generally, the adhesive is composed of an organic polymer different from the specific polymer contained in the particles, although quite commonly the adhesive represents a polymer containing many repeating units which are identical or similar to some of those present in the polymer composition of the particles.

Preferably, the adhesive is composed of one or more addition polymers formed from one or more ethylenically unsaturated polymerizable monomers, such as addition copolymers formed from two or more of such monomers. The adhesive can be prepared by any of a variety of conventional polymerization methods.

In addition to the above-described spread control and spreading zones, the element of the invention can also comprise any of a number of well known zones or combination of zones needed to determine, either qualitatively or quantitatively, the presence or amount of the analyte being tested. The exact choice and orientation of the various zones will vary according to the particular assay being performed and can easily be determined by one skilled in the art. For example, the element may comprise a reagent zone containing an indicator composition for providing a detectable change in response to the analyte being tested, and other well known optional zones such as registration zones for the indicator composition, radiation-blocking zones, subbing layers, etc. If an immunoassay is being performed, the element preferably comprises antibodies for the analyte immobilized, for example, on bacteria or on organo-polymeric particles.

The exact choice and orientation or configuration of the various reagent zones, spreading zones, registration zones, etc., of the elements of the invention can vary according to the assay being performed and will be known to one skilled in the art. Various elements for assays are described in the above-referenced U.S. Patents 3,992,158 and 4,258,001, and European Patent 66,648. Other elements having different configurations useful for a wide range of assays, which can utilize the present invention, are also well known in the art.

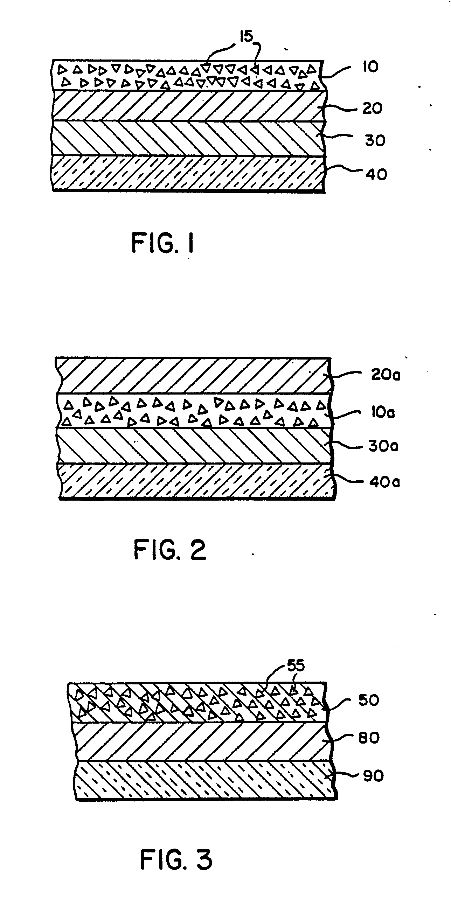

While the element of the invention can be of any configuration useful for performing any of a number of well known assays, reference to several exemplary configurations may be useful in describing the invention. FIGS. 1 and 2 illustrate elements of the invention, useful, for example, in an assay for glucose, each element having a spreading zone and a spread control zone where the spreading and spread control zones each comprise a separate layer of a multilayer analytical element. In FIG. 1, the spreading zone 10, spread control zone 20, and a reagent zone 30 are carried on a support 40. The spreading zone 10 is preferably a blushed polymer or particulate structure spreading zone as described in the above-referenced U.S. Patents 3,992,158 or 4,258,001. The spread control zone 20 comprises a polymeric material that is naturally soluble in water. Reagent zone 30 comprises a binder such as gelatin and an indicator composition interactive with the analyte. Support 40 can be any of a number of well known support materials, such as polyester or cellulose acetate.

FIG. 2 illustrates an alternative embodiment having a spread control zone 20a, spreading zone 10a and a reagent zone 30a, carried on support 40a. The spread control zone 20a comprises a water-soluble polymeric material. The spreading zone 10a is preferably a blushed polymer or particulate structure as described in U.S. Patents 3,992,158 or 4,258,001. Reagent zone 30a comprises a binder such as gelatin and an indicator composition. Support 40a can be any of the known support materials.

FIG. 3 represents a multilayer element of the invention having a spreading zone and a spread control zone where the spreading and spread control zones are contained in a single layer of the element. In FIG. 3, the spreading and spread control zones are contained in the particulate structure layer 50 having organo-polymeric carrier particles 55 (as described in U.S. Patent 4,258,001). Reagent zone 80 comprises a binder such as gelatin and an indicator composition interactive with the analyte. Support 90 can be any of a number of well known support materials, such as polyester or cellulose acetate.

The above-described elements according to the invention can be adapted for use in competitive immunoassays, end-point assays or rate assays.

An immunoassay can utilize any suitable label, for example, radioactive isotopes, fluorescers, enzymes, enzyme inhibitors and cofactors. Enzyme labels, such as glucose oxidase, peroxidase, and alkaline phosphatase are preferred.

When the elements are adapted to immunoassays, end-point assays or rate assays, an indicator composition is usually present in the element, although in some cases it can be added in the liquid sample or as a separate sample. This indicator composition can be a single component or a combination of reagents that can react with the analyte, a reaction product of the analyte or the label to produce a detectable change.

In one embodiment of this invention, the element is used for the determination of lactate dehydrogenase (LDH). The indicator composition useful in an LDH element is NADH (nicotinamide adenine dinucleotide) and sodium pyruvate.

The zones of the element can contain a variety of other desirable but optional components, including surfactants, thickeners, buffers, hardeners, antioxidants, coupler solvents, and other materials known in the art. The amounts of these components are also within the skill of a worker in the art.

When the spreading and spread control zones of the invention are separate layers in a multilayer analytical element, the element is prepared according to techniques well known in the art, such as described in the above-referenced U.S. Patents 3,992,158 and 4,258,001. The layer of water-soluble polymeric material can be coated as a layer in the element by well known means. When the spreading and spread control zones of the invention are contained in a single layer of an analytical element, a preferred method of preparing that layer is to first prepare a particulate spreading layer (such as described in U.S. Patent 4,258,001). Over that layer is coated a solution of a water-soluble polymer. This coating step is preferably performed so that the water-soluble polymer spreads into the spreading layer, coating the surface of the polymer particles. If the particles have antibodies immobilized thereon and the spread control zone is to contain a labeled antigen that is interactive with the antibodies, the coating is done in a way such that the antibodies and labeled antigen do not react. This is accomplished by a standard coating operation using the water-soluble polymers of this invention.

The elements of the invention are useful in performing any of a number of well known analyses, particularly analyses of biological fluids, such as end-point analyses, rate assays and homogeneous and heterogeneous immunoassays. The method of using the element of the invention will depend on the type of analysis being performed. Such methods are well known in the art and generally involve physically contacting a finite area of the element, preferably a spreading zone of the element, with an aqueous liquid sample (e.g., 1 to 200 μl) so that the liquid sample contacts the naturally water-soluble polymer in the element. The contacting is done in such a manner that the polymeric material in contact with the aqueous sample is dissolved, increasing the viscosity and reducing the spreading of the aqueous sample in the element. The contacting is done in the presence of an indicator composition for providing a detectable change in response to the presence of the analyte being tested. After contacting, the presence or amount of the analyte is determined as a function of the detectable change. Reagents can also be added simultaneously or sequentially with the liquid sample.

The contacting can be carried out by hand or with a machine using a pipette or other suitable dispensing means to dispense the test sample. If a heterogeneous immunoassay is being performed, it may be desirable to add a wash fluid after the liquid sample has been applied to the element. This wash causes unbound materials (e.g., analyte and labeled antigen) to move away from the bound materials. The wash fluid can contain a buffer and any other reagents that are described above, e.g., an enzyme substrate.

After sample application, the element is exposed to any conditioning, such as incubation, heating or the like, that may be desirable to quicken or otherwise facilitate obtaining the test result.

The following example is provided to illustrate the practice of the present invention.

As used in the context of this disclosure, I.U. represents the International Unit for enzyme activity defined as one I.U. being the amount of enzyme activity required to catalyze the conversion of 1 micromole of substrate per minute under standard pH and temperature conditions for the enzyme.

## Example

A test element for the determination of lactate dehydrogenase (LDH) is prepared as described in U.S. Serial No. 740,163 of Smith-Lewis, filed June 3, 1985. Except as otherwise noted quantities are parenthetically given in grams per square meter.

A poly(ethylene terephthalate) support is coated with a registration layer comprising hardened gelatin (5-20), Triton X-100 (trade mark) surfactant (0.05-1), N-tris(hydroxymethyl)methyl-2-aminoethane sulfonic acid (0.5-2.5), and NADH (0.1-0.8), and a spreading layer comprised of poly(vinyltoluene-co -p-t-butyl-styrene-co-methacrylic acid) (61:37:2 weight ratio) beads (100-200), poly-(N-isopropylacrylamide) (0.1-2), sodium pyruvate (0.01-2), N-tris(hydroxymethyl)methyl-2-aminoethane sulfonic acid (0.05-0.5), SURFLON S-132 (trade mark) fluoronated surfactant (0.1-10), and poly(vinyl-2-pyrrolidone) (0.1-2).

After these layers are applied, a coating of Klucel L (trade mark) hydroxypropylcellulose (0.05-5) is applied over the spreading layer so that the coating soaks into the spreading layer. After drying, the element is spotted with a 10 μl sample of control fluids containing four different concentrations of LDH (5 I.U./1; 1014 I.U./1; 1426 I.U./1; and 1904 I.U./1). The amount of LDH is then determined by measuring the decrease in reflection density at 340 nm with time at 37°C using a conventional spectrophotometer.

A control element lacking hydroxypropylcellulose is also prepared and tested as described above.

An enhanced reaction rate is observed with the test element indicating good spread control using hydroxypropylcellulose, i.e., less lateral spreading and less washout of small reagents.

## Claims

1. An analytical element for the determination of an analyte in an aqueous sample comprising a spread control zone comprising a polymeric material that is naturally soluble in water, wherein said polymeric material dissolves upon contact with the aqueous sample, thereby increasing the viscosity of the aqueous sample such that the spread of the aqueous sample in the element is reduced.

2. The element of claim 1 further comprising a porous spreading zone and a nonporous support.

3. The element of claim 2 wherein said porous spreading zone and spread control zone are contained in a single layer of said element.

4. The element of claim 2 wherein said porous spreading zone and spread control zone are each a separate layer of said element.

5. The element of claim 2, 3 or 4 further comprising a reagent zone containing an indicator composition for providing a detectable change in response to said analyte.

6. The element of claim 1 comprising multiple layers wherein said reduction in spreading is a reduction of lateral spreading in at least one of the layers of said element.

7. The element of any one of the preceding claims wherein said increase in viscosity is by a factor of at least 2-20.

8. The element of any one of the preceding claims wherein said polymeric material dissolves rapidly in water to the extent that a 0.5-25 μm thick layer of the polymeric material will dissolve in less than 5 minutes when contacted with water at less than 40°C on one side of the layer.

9. The element of any one of the preceding claims wherein the polymeric material is selected from the group consisting of unhardened gelatin, poly(vinylalcohol), poly(vinylpyrrolidone), and poly(acrylamide), or any mixtures or copolymers thereof, or from the group consisting of hydroxypropyl cellulose, hydroxyethyl cellulose, carboxymethyl cellulose, and carboxypropyl cellulose.

10. A method for the determination of an analyte component in an aqueous sample using an analytical element, comprising the steps of:

in the presence of an indicator composition for providing a detectable change in response to said analyte, contacting a finite area of the element with said aqueous sample; and

determining the presence or amount of said analyte as a function of the detectable change in said indicator composition,

the spread of said aqueous sample in the element being controlled by contacting the aqueous sample with a polymeric material that is naturally soluble in water, in such a manner that the polymeric material in contact with the aqueous sample is dissolved, whereby the viscosity of the aqueous sample is increased and the spreading of the aqueous sample in the element is reduced.

FIG. I

FIG. 2

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | US-A-4 547 465 (J.N. EIKENBERRY) <br> * Column 2, lines 26-63; column 13, lines 31-39; column 14, lines 15-27; column 17, claim 16 * <br> --- | 1-6,10 | G 01 N 33/52 |
| X | US-A-4 361 648 (C. SHUENN-TZONG) <br> * Column 1, line 61 - column 2, line 25; column 4, lines 48-51 * <br> --- | 1-6,9, 10 | |
| Y | DE-A-2 739 008 (BEHRINGWERKE AG) <br> * Page 1, claims 1,2; page 4, lines 20-33; page 11, lines 17-26 * <br> --- | 1-6,9, 10 | |
| Y | US-A-4 166 093 (M.J. SMITH-LEWIS et al.) <br> * Column 3, lines 6-43; column 5, lines 47-54 * <br> --- | 1-6,9, 10 | TECHNICAL FIELDS SEARCHED (Int. Cl.4) <br><br> G 01 N 33/00 <br> G 01 N 21/00 <br> G 01 N 35/00 |
| Y | PATENT ABSTRACTS OF JAPAN, vol. 10, no. 152 (P-462)[2208], 3rd June 1986; & JP-A-61 4963 (FUJI SHASHIN FILM K.K.) 10-01-1986 <br><br> ----- | 1-6,9, 10 | |

. The present search report has been drawn up for all claims

| Place of search | Date of completion of the search | Examiner |
|---|---|---|
| THE HAGUE | 27-10-1987 | VAN BOHEMEN C.G. |